# EUROPEAN PATENT SPECIFICATION

(11) **EP 3 102 559 B1**
(45) Date of publication and mention of the grant of the patent: **07.08.2019**
(21) Application number: 15705530.2
(22) Date of filing: 05.02.2015
(51) Int. Cl.: C07C 209/36, C07C 211/50, B01J 19/18

(54) **METHOD FOR HYDROGENATION OF AROMATIC NITROCOMPOUNDS FOR THE PRODUCTION OF AROMATIC AMINES, AND A SYSTEM THEREFORE**
VERFAHREN ZUR HYDRIERUNG AROMATISCHER NITROVERBINDUNGEN ZUR HERSTELLUNG VON AROMATISCHEN AMINEN UND EIN SYSTEM DAFÜR
PROCÉDÉ POUR L'HYDROGÉNATION DE COMPOSÉS NITRO AROMATIQUES POUR OBTENIR DES AMINÉS AROMATIQUES ET SYSTÈME ASSOCIÉ

(30) Priority: 06.02.2014 SE 1450124
(43) Date of publication of application: 14.12.2016
(73) Proprietor: Chematur Technologies AB, 691 27 Karlskoga (SE)
(72) Inventor: STENMARK, Lars, 691 41 Karlskoga (SE)
(74) Representative: AWA Sweden AB
(86) International application number: PCT/EP2015/052392
(87) International publication number: WO 2015/118059

(56) References cited:
- EP-A1- 2 471 768
- CN-A- 101 205 189
- CN-A- 102 952 022
- US-A1- 2012 289 745

## Description

### Technical field

The present invention relates to a continuous process for the preparation of aromatic amines by catalytic hydrogenation of the corresponding nitrocompounds on which the amines are based.

### Background of the invention

Catalytic hydrogenation of aromatic nitrocompounds to produce the corresponding amines in a continuous mode is a very important industrial process. Examples of such processes are the hydrogenation of nitrobenzene (NB) to produce aniline (AN) and the hydrogenation of dinitrotoluene (DNT) to produce toluenediamine (TDA). In the preparation of aromatic amines by reaction of nitro compounds with hydrogen, which is known in the art, a considerable amount of heat is liberated.

The catalyst used in the hydrogenation process may for example be a precious metal, such as palladium (Pd) or platinum (Pt) on a carrier or a nickel based catalyst.

A well known and convenient manner of carrying out a hydrogenation process in liquid phase is to use a well agitated continuous stirred tank reactor (CSTR) with continuous addition of the feed nitrocompound and hydrogen and continuous recovery of the obtained product mixture. The catalyst present in the process is filtered off and returned to the hydrogenator.

For large scale processes it is in many cases advantageous not to use a solvent in the process since that would involve further process steps like distillation to remove, purify and recycle the solvent in order to obtain a pure product. Instead the product mixture itself is used as the solubilising medium as a CSTR by definition always is operated at the exit conditions. The average residence time of the components in the CSTR is typically several hours.

During a continuous hydrogenation process it can be difficult to achieve a complete hydrogenation of the feed material and the intermediates as new material of the feed is constantly being added to a single CSTR hydrogenator.

Due to the continuous manner there is always a risk that a small portion of the feed material is experiencing too short residence time in the well agitated reaction tank to be completely hydrogenated. A normal counter measure to minimise any residual feed material and any intermediates is to increase the added amount of catalyst, increase the residence time, increase the hydrogen pressure and/or the reaction temperature in order to achieve a higher reaction rate and in that manner optimize product output and contamination from feed material and intermediates. A drawback with this counter measure is that there is a risk that the product becomes over hydrogenated and/or that some unwanted side reaction occurs. The side reactions which frequently result in the formation of interfering substances, for example tar-like constituents, and thus in reduction of the yield, are a problem in many processes used hitherto.

Other ways to increase the product output is to use several reactors in series. It is known to use reactors in series which are fed fresh hydrogen to drive the reaction further towards a more complete hydrogenation. A post-reactor for completion of the reaction can be located between the reaction mixture outlet and a subsequent separation unit. Such a post-reactor can have the same design as the main reactor. A stirred reactor or a flow tube can be used as a post reactor.

Also, when performing a hydrogenation process, if the reaction is driven too far this could result in the ingoing components reacting further and forming undesirable byproducts leading to a decreased yield of desirable products.

CN102952022 discloses a method for preparing diamino toluene from dinitrotoluene with a catalyst slurry comprising palladium. After hydrogenation of the feed material, the material is forwarded to a tube reactor for further reaction, via a venturi mixer in which additional hydrogen is added.

US2012/0289745 discloses a process for the preparation of aromatic amines in liquid phase by catalytic hydrogenation of the corresponding nitroaromatic compounds in at least two reaction spaces connected in series, wherein at least one reaction space is operated isothermally and at least the reaction space connected downstream thereof is operated adiabatically.

Thus, there is a need for new possibilities to maintain or improve the obtained product quality and output amount. Also, there is a need for simpler and more economical processes and systems.

### Summary of the invention

The present invention relates to providing a method of increasing the output of a high quality product without the need for further input of any fresh feed material. By performing the hydrogenation in subsequent reactors equipped with specific distribution means a full hydrogenation and high quality end product is obtained.

The present invention overcomes the drawbacks of known methods and systems by introduction of a polishing reactor after a main hydrogenation reactor. The overflow from the main hydrogenation reactor enters into a polishing reactor, which may be considerably smaller in size. The polishing reactor is equipped with a gas recirculation device, which recirculates and distributes the gaseous phase containing hydrogen in the overflowing liquid, the liquid phase. As there is no addition of fresh feed material into the overflow liquid the residual small amounts of feed material and intermediates present will be completely hydrogenated.

An object of the present invention is to provide a process for the preparation of amines by hydrogenation of aromatic nitrocompounds in liquid phase, which comprises supplying a first hydrogenation reactor comprising at least one catalyst with a feed of gaseous hydrogen and a feed of liquid aromatic nitrocompounds; hydrogenation in the first reactor by contacting the gaseous phase with the liquid phase to obtain a product containing aromatic amines; forwarding the liquid and gaseous phases present in the first reactor to a second polishing reactor; and contacting gaseous phase material present in the polishing reactor with the liquid phase to allow further hydrogenation of any remaining aromatic nitrocompounds and/or intermediates; wherein the polishing reactor is only fed material from the first reactor.

According to one embodiment the polishing reactor includes a step of gas recirculation and distribution of the gaseous phase to the liquid phase.

According to one embodiment the polishing reactor comprises an agitating device which has a hollow stirrer shaft, being arranged for gas recirculation and distribution of the gaseous phase to the liquid phase.

According to one embodiment the polishing reactor comprises a recirculation line for gaseous phase which forwards gaseous phase present in the upper part of the polishing reactor and introducing said gaseous phase below a gassed liquid level of the polishing reactor, preferably at a position below the agitation device.

According to one embodiment the process further comprises a step of disengagement of gaseous phase from the liquid phase.

According to one embodiment the process further comprises a separation step for separating the prepared amines from the at least one catalyst present during the hydrogenation.

An object of the present invention is to provide use of a system for the preparation of amines by hydrogenation of aromatic nitrocompounds in liquid phase, which comprises
a hydrogenation reactor,
a first agitating device arranged in the first reactor,
an inlet for nitroaromatic compounds arranged in the first reactor,
an inlet for hydrogen arranged in the first reactor,
a polishing reactor, having at most 1/20 of the volume of the hydrogenation reactor,
a connecting device connecting said hydrogenation and polishing reactors, which is an inlet for said polishing reactor, and an outlet for said hydrogenation reactor,
wherein the connecting device is the only inlet for said polishing reactor and is arranged adjacent to or at about a gassed liquid level of the reactors.

According to one embodiment the polishing reactor includes arrangements for gas recirculation and distribution.

According to one embodiment the polishing reactor comprises a recirculation line for gaseous phase connected to the upper part of the polishing reactor and below a gassed liquid level of the polishing reactor, preferably at a position below the agitation device.

According to one embodiment the polishing reactor comprises an agitation device which has a hollow stirrer shaft, being arranged for gas recirculation and distribution.

According to one embodiment the system further comprises a disengagement device subsequent of the polishing reactor.

According to one embodiment the system further comprises a filter.

### Short description of the drawings

Figure 1 shows a set-up of a reactor system for the production of aromatic amines from aromatic nitrocompounds with an internal gas recirculation device.
Figure 2 shows a set-up of a reactor system for the production of aromatic amines from aromatic nitrocompounds with an external gas recirculation device.

In the figures, the following units or parts are presented with reference numbers.
- 1: Hydrogenation system
- 2: Main hydrogenation reactor
- 3: Agitation device 1
- 4: Stirrer shaft
- 5: Upper agitator
- 6: Lower agitator
- 7: Agitation device motor
- 8: Inlet nitroaromatic compounds
- 9: Inlet hydrogen
- 10: Cooling device
- 11: Connecting means
- 12: Gassed liquid level
- 13: Polishing reactor
- 14: Agitation device 2
- 15: Agitator
- 16: Stirrer shaft
- 17: Agitation device motor
- 18: Disengagement vessel
- 19: Pump
- 20: Filter
- 21: Product outlet
- 22: Recirculation line for catalyst slurry
- 23: Recirculation line for gaseous phase
- 24: Forwarding device

The figures only represent possible specific embodiments of the different aspects of the invention and they should only be viewed as examples.

### Detailed description

The present process involves reaction of aromatic nitrocompounds and hydrogen.

Ingoing feed materials may be aromatic nitrocompounds having one or more nitro groups and 6 to 18 carbon atoms, e.g. nitrobenzenes, e.g. nitrobenzene, 1, 3-dinitrobenzene, nitrotoluenes, for example 2, 4- or 2,6-dinitrotoluene, 2,4,6-trinitrotoluene, nitroxylenes, for example 1,2-dimethyl-3-, 1,2-dimethyl-4-, 1, 4-dimethyl-2-, 1,3-dimethyl-2-, 2,4-dimethyl-1- and 1,3-dimethyl-5-nitrobenzene, nitronaphthalenes, e.g. 1-, 2-nitronaphthalene, 1,5- and 1,8-dinitronaphthalene, chloronitrobenzenes, e.g. 2-chloro-1, 3- and 1-chloro-2, 4-dinitrobenzene, o-, m- and p-chloronitrobenzene, 1, 2-dichloro-4-, 1,4-dichloro-2-, 2,4-dichloro-1- and 1,2-dichloro-3-nitrobenzene, chloro-nitrotoluenes, e.g. 4-chloro-2, 4-chloro-3-, 2-chloro-4- and 2-chloro-6-nitrotoluene, nitroanilines, e.g. o-, m- and p-nitroaniline; nitroalcohols, e.g. tris(hydroxymethyl)-nitromethane, 2-nitro-2-methyl- and 2-nitro-2-ethyl-1, 3-propanediol, 2-nitro-1-butanol and 2-nitro-2-methyl-1-propanol, and any desired combination thereof.

As an example the nitrocompound feed may include nitrobenzene, nitrotoluene or dinitrotoluene, e.g. 2,4-dinitrotoluene and/or 2,6-dinitrotoluene.

The present process is particularly advantageous in the hydrogenation of dinitrotoluene to the corresponding toluenediamine (TDA) and in the hydrogenation of nitrobenzene to aniline. Most preferred it is to use the present process in the hydrogenation of dinitrotoluene.

The preparation of aromatic amines according to the present method is carried out at the usual temperatures and pressures for the preparation of aromatic amines.

The hydrogenation of the nitroaromatic compounds to the corresponding amines takes place with hydrogen or mixtures of hydrogen and inert gases as the hydrogenation reagent. Suitable catalysts are all catalysts conventional for catalytic hydrogenations. Catalysts that contain noble metals such as Pt, Pd, Rh, Ru or non-ferrous metals such as Ni, Co or Cu or mixtures thereof are preferably used. Catalysts that contain Pt, Pd, Ni or Cu are particularly preferably used, namely in the form of a suspension in water. In the case of noble metal catalysts, they are applied to supports such as, for example, carbon black, activated carbon, SiO₂ or Al₂O₂.

The present method and use of the system involves a hydrogenation reaction which takes place in a first main hydrogenation reactor which is connected by connecting means to a second polishing reactor. The second reactor may be considerably smaller than the first reactor. In the first reactor the main part of the hydrogenation occurs and any remaining nitrocompound feed material and/or intermediates are hydrogenated in the subsequent polishing reactor. The main hydrogenation reactor and the polishing reactor are connected by connecting means. The connecting means may be in the form of at least one pipeline. The connecting means may forward both liquid phase and gaseous phase in one and the same pipeline, from the first to the second reactor. Further, an additional pipeline connecting the gaseous phase, from the first to the second reactor may be used, to ensure pressure equalization between the two reactors. The two reactors are arranged so that the connecting means are forwarding both liquid phase and gaseous phase between the reactors. Preferably the connecting means are arranged adjacent or about the gassed liquid level of the reactors. The gassed liquid level is at about the same level in both reactors and the connecting means is a device connecting the reactors and allowing liquid phase in the first reactor to be forwarded continuously to the second reactor via said connecting means. The gassed liquid level in the second reactor may be controlled at a level slightly below the connecting pipe to ensure that the overflow from the first reactor by gravity is always possible. In a similar manner also the gaseous phase is forwarded from one reactor to the other. In the polishing reactor the gaseous phase containing unreacted hydrogen from the first main reactor is brought into contact with any remaining nitrocompound feed material and/or intermediates of the liquid phase of the polishing reactor. Any catalyst used for the hydrogenation process is preferably added only to the first main hydrogenation reactor. The polishing reactor is only fed material from the preceding reactor. No fresh material or other additional materials are then added from other sources. Both gaseous and liquid material is forwarded from the preceding reactor to the polishing reactor. Thus, the polishing reactor only receive any remaining catalysts in the feed from the first main hydrogenation reactor. The hydrogen gas in the polishing reactor may be brought in contact with any remaining nitrocompound feed material and/or intermediates of the liquid phase via a gas recirculation device. The gas recirculation device takes and transports gaseous phase comprising hydrogen and contacts it with the liquid phase. The gas recirculation device may be an external or internal device of the polishing reactor.

As an external gas recirculation device gaseous phase may be withdrawn from the gas containing part of the polishing reactor and/or the gas containing part of the preceding reactor, and the gaseous phase may then be reintroduced and/or introduced into the polishing reactor in the liquid containing part of the polishing reactor. The recirculation may be facilitated by the use of a forwarding device such as compressor(s), pump(s), fan(s), e.g. blowing fans etc. The introduction of gaseous phase into the liquid phase of the polishing reactor may occur anywhere in the liquid phase of the polishing reactor. However, it is preferable if the introduction is close to an agitation device which aids the distribution of the gaseous phase throughout the liquid phase. Preferably, the gaseous phase is introduced at a position below the agitator in the polishing reactor to ensure maximal efficiency. Preferably, the gaseous phase is introduced at a position in the vicinity of the agitator.

The gas recirculation device may contain a distribution section, e.g. a sparger, spreader, diffuser, etc, to facilitate the distribution of the gaseous phase in the liquid phase.

In one embodiment, present in the polishing reactor is preferably an agitation device, as disclosed above. The agitation device is run by a motor. The agitation device is constructed in such a manner that the motor is connected to a stirrer shaft, which in turn is connected to an agitator. The agitator is the device in the fluid which effects the mixing, and may include baffles and propeller or turbine blades etc.

Alternatively, the gas recirculation device may be an internal device of the polishing reactor. The agitation device may be arranged for gas recirculation and distribution. The stirrer shaft may be a hollow shaft with holes which are placed in the gas space above the gassed liquid level of the polishing reactor. Through these holes hydrogen may be transported within the stirrer shaft down to a distribution section of the agitation device which is placed in the liquid space below the gassed liquid level of the polishing reactor, and from which the hydrogen is forwarded or fed into the liquid phase. In the lower section of the agitation device which includes the agitator, the hollow shaft and/or said agitator may contain a distribution section. The distribution section is in the vicinity of the agitator. Said distribution section may include openings or holes of the hollow shaft adjacent to the agitator (such as the blades of the agitator). Further, parts of the agitator, such as the blades of the agitator may be hollow. Thus, said distribution section may include openings or holes of the agitator (such as openings or holes in the blades of the agitator). According to a preferred embodiment the openings or holes of the distribution section may be placed at the outer part of the agitator blades, i.e. the outer edge at a distance from the shaft (in the periphery of the blades). More preferably said openings or holes are placed on the back or rear side of the blade, in relation to the rotation direction when the agitator is in use. When the agitator is rotating, this arrangement will create a pumping effect for the gaseous phase, enabling efficient gas recirculation. With the construction of a hollow shaft stirrer, hydrogen and nitrocompounds and/or intermediates may be contacted with each other to ensure a complete hydrogenation of any nitrocompounds and/or intermediates of the reaction or system.

Furthermore, the extent of reaction in the polishing reactor, i.e. the polishing reaction, that is achieved can be optimised by altering the intensity of agitation in the reactor. The higher the agitation speed, the higher the rate of polishing (i.e. final conversion of any feed material or intermediates present). Thus, by the present method and use of the present system one may hydrogenate residual feed material and intermediates without excessive side reactions.

The polishing reactor is preferably considerably smaller in size compared to the main hydrogenation reactor. The difference in reaction volume between the two may e.g. be at most 1:20, i.e. the polishing reactor is at most 1/20 of the volyme of the main hydrogenation reactor. In other embodiments the polishing reactor is at most 1/25, e.g. at most 1/30, at most 1/35, at most 1/40, or at most 1/50, of the volyme of the main hydrogenation reactor.

The residence time in the polishing reactor, where the reaction occurs, is normally short. The residence time may be a few minutes, e.g. from less than one minute up to 10 minutes, such as 0.5 - 7 minutes , 0.5 - 5 minutes, depending on the application.

The embodiments of the present invention allow a difference in size between the main hydrogenation reactor and the subsequent the polishing reactor. Connected to the above disclosed is also the ability to achieve a short residence time. The contacting of gaseous phase material with the liquid phase material of the polishing reactor achieves a desired hydrogenation.

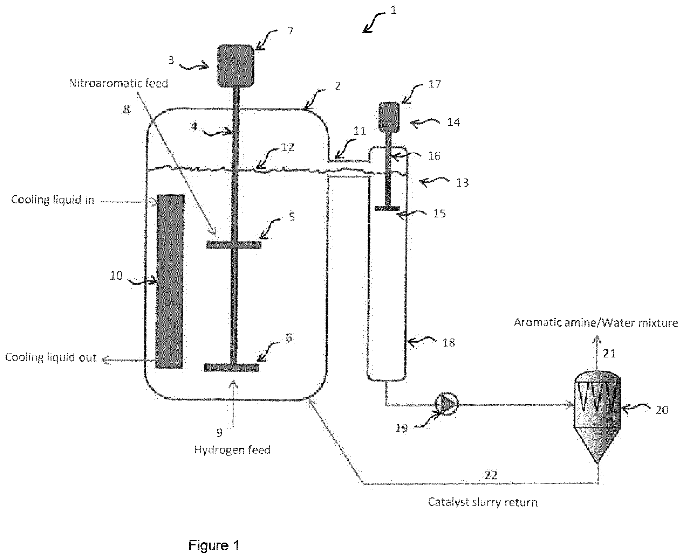

Figure 1 shows a setup of the present invention. The disclosed hydrogenation system 1 comprises a main hydrogenation reactor 2, e.g. a continuous stirred tank reactor (CSTR) hydrogenator, with a cooling device 10, a first agitation device 3, connecting means 11, a second reactor being a polishing reactor 13, and a second agitation device 14.

The first agitation device 3 comprises an agitation device motor 7, a stirrer shaft 4, an upper agitator 5 and a lower agitator 6, preferably located close to the bottom of the reactor. The stirrer shaft 4 can be made as a hollow shaft with holes drilled in the gas space above the gassed liquid level 12 and corresponding holes at the upper agitator 5. If the agitator is correctly designed this set-up will enable an internal recirculation of the gaseous phase into the liquid phase to ensure an intimate mixing of the hydrogen gas with the liquid containing the nitrocompound to be hydrogenated. Fresh hydrogen is typically introduced at the bottom part of the hydrogenation reactor via a hydrogen inlet 9 and dispersed. Dispersing of the hydrogen may be performed by the lower agitator 6. The inlet 9 of fresh hydrogen may be located on the lower agitator 6 or adjacent to the lower agitator 6. Fresh hydrogen is introduced at a rate sufficient to keep the operating pressure constant at the desired level. At or adjacent to the mentioned upper agitator 5 the fresh aromatic nitrocompounds may be introduced into the main hydrogenation reactor 2 via an inlet 8.

The second agitation device 14 comprises an agitation device motor 17, a stirrer shaft 17, and an agitator 15. As mentioned for the first agitation device 3 above, the second agitation device 14 also have a stirrer shaft 16 that can be made as a hollow shaft with holes or openings placed in the gas space above the gassed liquid level and corresponding holes/openings at the agitator 15. Thus, it is also here achieved an internal recirculation of the gaseous phase into the liquid phase to ensure an intimate mixing of the hydrogen gas with the liquid containing the any remaining aromatic nitrocompounds or intermediates to be hydrogenated. It is preferable that the openings or holes present on the agitation device 14 to be in the liquid space below the gassed liquid level are placed on the agitator 15, such as on the blades of the agitator 15, and more preferably said openings or holes are located behind, i.e. at the backside of, the blades with respect to the rotation direction of the agitator 15, in order to create a lower pressure where the holes exits, enabling the gas recirculation from the gas phase.

The hydrogenation reaction is very exothermic and the heat of reaction is removed by means of cooling liquid by internal or external heat exchangers or a combination of the two. If possible it is advantageous to use the excess heat to produce e.g. steam by operating the reaction at high enough reaction temperature or upgrade the heat energy for such processes.

The formed product mixture is constantly overflowing via the connecting means 11 into the polishing reactor 13. The connecting means 11 may also include an additional stream (not shown) of gaseous phase between the main hydrogenation reactor 2 and the polishing reactor 13 to ensure pressure equalization. In the polishing reactor 13 any remaining aromatic nitrocompounds and intermediates are reacted with hydrogen present in the gaseous phase above the gassed liquid level 12, as hydrogen gas is forwarded down into the liquid phase via the hollow stirrer shaft 16 and distributed by the agitator 15. In this manner any residual nitrocompounds and intermediates are converted into the desired end products, the aromatic amines. Also the process is run at the same conditions as the main hydrogenation reactor due to the open character of the connecting means. After the polishing reactor 13, the formed product mixture is forwarded to a disengagement vessel 18 where the hydrogen gas bubbles are separated from the liquid to enable pumping of the product slurry without cavitation. The disengagement also means that the catalytic hydrogenation will stop due to lack of hydrogen and poor mass transfer from gas to liquid phase. The polishing reactor 13 and disengagement vessel 18 may be constructed as separate units, one downstream of the other, or they may be separate parts of one single apparatus unit, just subsequent parts of each other, within the same apparatus construction. From the bottom of the disengagement vessel 18 the mixture is pumped via a pump 19, e.g. a catalyst thickener feed pump, to a filter 20, e.g. a catalyst thickener filer, where the catalyst is retained and returned to the main hydrogenation reactor 2 via recirculation line 22 and the catalyst free product mixture, containing the amine and the water produced in the reaction, is sent downstream for further processing via line 21.

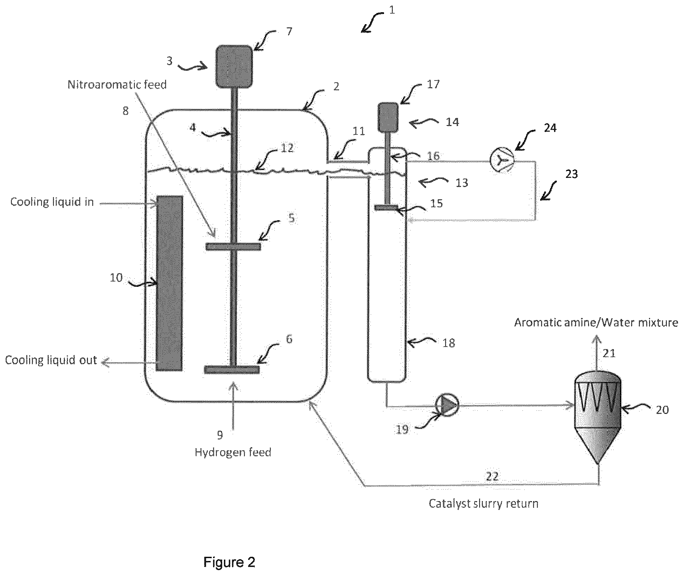

Figure 2 shows a setup of the present invention. The embodiment disclosed in figure 2 is similar to the one disclosed in figure 1 except for that the gas recirculation device of figure 1 is internal and involves a hollow shaft agitator and in figure 2 the gas recirculation device is external without a hollow shaft agitator. Thus the main part is similar as disclosed for figure 1. In figure 2 the gas recirculation is performed using a recirculation line for gaseous phase 23, which is facilitated by a forwarding device 24. It is preferable if the recirculation line 23 enters the polishing reactor 13 at a position below the agitator 15 of the agitation device 14.

### Examples

Tests were made to see the effect of including a polishing reactor after a main continuous stirred tank reactor (CSTR). The tests were performed for a continuous hydrogenation of DNT to produce TDA.

The different trial numbers represents different trials carried out a temperature of 115 °C and 8.5 bar system pressure. A proprietary catalyst based on Pd and Pt on an activated carbon carrier was used. The only difference between the trials was the type of activated carbon carrier which was used. In these examples the polishing time was about 1 minute at intensive mixing conditions.

| Test | Trial | Sample point | TDA (%) | DNT (%) | Intermediates (%) | Tar (%) |
|---|---|---|---|---|---|---|
| 1 | A | content in CSTR | 95.97 | 0.0102 | 0.0388 | 0.93 |
| 2 | A | content after polishing | 96.16 | 0 | 0 | 0.79 |
| 3 | B | content in CSTR | 96.24 | 0.0037 | 0.014 | 0.92 |
| 4 | B | content after polishing | 96.43 | 0 | 0 | 0.78 |
| 5 | C | content in CSTR | 96.4 | 0.0021 | 0.0099 | 0.87 |
| 6 | C | content after polishing | 96.51 | 0 | 0 | 0.76 |

The data obtained from the tests clearly shows a benefit of using a polishing step. After the main hydrogenation step in the CSTR, there still remains some nitrocompound feed material and intermediates in the product mixture. After the polishing step, no nitrocompound feed materal or intermediates are present in the product mixture with an increased yield of product as a result. Of even more importance is that the purity of the product after polishing is much improved making it more suitable for use as raw material for production of downstream products.

## Claims

1. A process for the preparation of amines by hydrogenation of aromatic nitrocompounds in liquid phase, which comprises supplying a first hydrogenation reactor (2) comprising at least one catalyst with a feed (9) of gaseous hydrogen and a feed (8) of liquid aromatic nitrocompounds; hydrogenation in the first reactor (2) by contacting the gaseous phase with the liquid phase to obtain a product containing aromatic amines; forwarding the liquid and gaseous phases present in the first reactor (2) to a second polishing reactor (13); and contacting gaseous phase material present in the polishing reactor (13) with the liquid phase to allow further hydrogenation of any remaining aromatic nitrocompounds and/or intermediates,
wherein the polishing reactor (13) is only fed material from the first reactor.

2. The process according to claim 1, wherein the polishing reactor (13) includes a step of gas recirculation and distribution of the gaseous phase to the liquid phase.

3. The process according to claim 1 or 2, wherein the polishing reactor (13) comprises an agitating device (14) which has a hollow stirrer shaft (16), being arranged for gas recirculation and distribution of the gaseous phase to the liquid phase.

4. The process according to claim 1 or 2, wherein the polishing reactor (13) comprises a recirculation line for gaseous phase (23) which forwards gaseous phase present in the upper part of the polishing reactor (13) and introducing said gaseous phase below a gassed liquid level (12) of the polishing reactor (13), preferably at a position below the agitation device (14).

5. The process according to anyone of claims 1-4, further comprising a step of disengagement of the gaseous phase from the liquid phase.

6. The process according to anyone of claims 1-5, further comprising a separation step for separating the prepared amines from the at least one catalyst present during the hydrogenation.

7. Use of a system which comprises
a hydrogenation reactor (2),
an first agitating device (3) arranged in the first reactor (2),
an inlet (8) for nitroaromatic compounds arranged in the first reactor (2),
an inlet (9) for of hydrogen arranged in the first reactor (2),
a polishing reactor (13), having at most 1/20 of the volume of the hydrogenation reactor (2),
a connecting device (11), connecting said hydrogenation and polishing reactors (2,13), which is an inlet for said polishing reactor (13), and an outlet for said hydrogenation reactor (2),
wherein the connecting device (11) is the only inlet for said polishing reactor (13) and is arranged adjacent to or at about a gassed liquid level of the reactors (2,13)
for the preparation of amines by hydrogenation of aromatic nitrocompounds in liquid phase as claimed in claim 1.

8. The use according to claim 7, wherein the polishing reactor (13) includes arrangements for gas circulation and distribution.

9. The use according to claims 7 or 8, wherein the polishing reactor (13) comprises an agitation device (14) which has a hollow stirrer shaft (16), being arranged for gas recirculation and distribution.

10. The use according to claims 7 or 8, wherein the polishing reactor (13) comprises a recirculation line for gaseous phase (23) connected to the upper part of the polishing reactor (13) and below a gassed liquid level (12) of the polishing reactor (13), preferably at a position below the agitation device (14).

11. The use according to anyone of claims 7-10, further comprising a disengagement device (18) subsequent of the polishing reactor (13).

12. The use according to anyone of claims 7-11, further comprising a filter (20).

## Patentansprüche

1. Verfahren zur Herstellung von Aminen durch Hydrierung aromatischer Nitroverbindungen in flüssiger Phase, das ein Zuführen von gasförmigem Wasserstoff als Einsatzmaterial (9) und von flüssigen aromatischen Nitroverbindungen als Einsatzmaterial (8) in einen ersten Hydrierungsreaktor (2), der mindestens einen Katalysator umfasst; eine Hydrierung im ersten Reaktor (2) durch In-Kontakt-Bringen der gasförmigen Phase mit der flüssigen Phase zum Erhalten eines Produkts, das aromatische Amine enthält; ein Weiterbefördern der im ersten Reaktor (2) vorhandenen flüssigen und gasförmigen Phase zu einem zweiten Nachbehandlungsreaktor (13) und ein In-Kontakt-Bringen von in dem Nachbehandlungsreaktor (13) vorhandenen Material in gasförmiger Phase mit der flüssigen Phase zur Ermöglichung einer weiteren Hydrierung verbleibender aromatischer Nitroverbindungen und/oder Zwischenprodukte umfasst,
wobei dem Nachbehandlungsreaktor (13) nur Material aus dem ersten Reaktor zugeführt wird.

2. Verfahren nach Anspruch 1, wobei der Nachbehandlungsreaktor (13) einen Schritt zur Gasrückführung und Verteilung der gasförmigen Phase in der flüssigen Phase umfasst.

3. Verfahren nach Anspruch 1 oder 2, wobei der Nachbehandlungsreaktor (13) eine Rührvorrichtung (14) umfasst, die eine hohle Rührwelle (16) aufweist und zur Gasrückführung und Verteilung der gasförmigen Phase in der flüssigen Phase angeordnet ist.

4. Verfahren nach Anspruch 1 oder 2, wobei der Nachbehandlungsreaktor (13) eine Rückführungsleitung für die gasförmige Phase (23) umfasst, in der die im oberen Teil des Nachbehandlungsreaktors (13) vorliegende gasförmige Phase weiterbefördert wird und mit der die gasförmige Phase unter einem Füllstand von mit Gas versetzter Flüssigkeit (12) des Nachbehandlungsreaktors (13), vorzugsweise an einer Position unter der Rührvorrichtung (14), eingeleitet wird.

5. Verfahren nach einem der Ansprüche 1 bis 4, ferner umfassend einen Schritt zum Trennen der gasförmigen Phase von der flüssigen Phase.

6. Verfahren nach einem der Ansprüche 1 bis 5, ferner umfassend einen Abtrennschritt zum Abtrennen der hergestellten Amine von dem mindestens einen während der Hydrierung vorhandenen Katalysator.

7. Verwendung eines Systems, das umfasst:
einen Hydrierungsreaktor (2),
eine erste Rührvorrichtung (3), die in dem ersten Reaktor (2) angeordnet ist,
einen Zulauf (8) für nitroaromatische Verbindungen, der im ersten Reaktor (2) angeordnet ist,
einen Zulauf (9) für Wasserstoff, der im ersten Reaktor (2) angeordnet ist,
einen Nachbehandlungsreaktor (13), der höchstens 1/20 des Volumens des Hydrierungsreaktors (2) aufweist,
eine den Hydrierungs- und den Nachbehandlungsreaktor (2, 13) verbindende Verbindungsvorrichtung (11), die ein Zulauf für den Nachbehandlungsreaktor (13) und ein Ablauf für den Hydrierungsreaktor (2) ist,
wobei die Verbindungsvorrichtung (11) der einzige Zulauf für den Nachbehandlungsreaktor (13) ist und angrenzend an einen oder bei ungefähr einem Füllstand der mit Gas versetzten Flüssigkeit der Reaktoren (2, 13) angeordnet ist,
zur Herstellung von Aminen durch Hydrierung aromatischer Nitroverbindungen in flüssiger Phase nach Anspruch 1.

8. Verwendung nach Anspruch 7, wobei der Nachbehandlungsreaktor (13) Anordnungen zur Gasrückführung und -verteilung aufweist.

9. Verwendung nach Anspruch 7 oder 8, wobei der Nachbehandlungsreaktor (13) eine Rührvorrichtung (14) umfasst, die eine hohle Rührwelle (16) aufweist und zur Gasrückführung und -verteilung angeordnet ist.

10. Verwendung nach Anspruch 7 oder 8, wobei der Nachbehandlungsreaktor (13) eine Rückführungsleitung für die gasförmige Phase (23) umfasst, die mit dem oberen Teil des Nachbehandlungsreaktors (13) verbunden und unterhalb eines Füllstands von mit Gas versetzter Flüssigkeit (12) des Nachbehandlungsreaktors (13), vorzugsweise an einer Position unter der Rührvorrichtung (14), angeschlossen ist.

11. Verwendung nach einem der Ansprüche 7 bis 10, die ferner eine Trennvorrichtung (18) im Anschluss an den Nachbehandlungsreaktor (13) umfasst.

12. Verwendung nach einem der Ansprüche 7 bis 11, die ferner einen Filter (20) umfasst.

## Revendications

1. Procédé de préparation d'amines par hydrogénation de nitrocomposés aromatiques en phase liquide, qui comprend l'alimentation d'un premier réacteur d'hydrogénation (2) comprenant au moins un catalyseur avec une matière première (9) d'hydrogène gazeux et une matière première (8) de nitrocomposés aromatiques liquides ; hydrogénation dans le premier réacteur (2) par mise en contact de la phase gazeuse avec la phase liquide pour obtenir un produit contenant des amines aromatiques ; transfert des phases liquide et gazeuse présentes dans le premier réacteur (2) vers un deuxième réacteur de polissage (13) ; et mise en contact du matériau en phase gazeuse présent dans le réacteur de polissage (13) avec la phase liquide pour permettre l'hydrogénation supplémentaire de nitrocomposés aromatiques et/ou intermédiaires restants, dans lequel le réacteur de polissage (13) est alimenté uniquement avec un matériau du premier réacteur.

2. Procédé selon la revendication 1, dans lequel le réacteur de polissage (13) comprend une étape de recirculation et distribution de gaz de la phase gazeuse vers la phase liquide.

3. Procédé selon la revendication 1 ou 2, dans lequel le réacteur de polissage (13) comprend un dispositif d'agitation (14) qui comporte un arbre d'agitation creux (16), étant agencé pour recirculation et distribution de gaz de la phase gazeuse vers la phase liquide.

4. Procédé selon la revendication 1 ou 2, dans lequel le réacteur de polissage (13) comprend une conduite de recirculation pour la phase gazeuse (23) qui transfère la phase gazeuse présente dans la partie supérieure du réacteur de polissage (13) et introduisant ladite phase gazeuse au-dessous d'un niveau de liquide gazéifié (12) du réacteur de polissage (13), de préférence à une position au-dessous du dispositif d'agitation (14).

5. Procédé selon l'une quelconque des revendications 1 à 4, comprenant en outre une étape de dissociation de la phase gazeuse à partir de la phase liquide.

6. Procédé selon l'une quelconque des revendications 1 à 5, comprenant en outre une étape de séparation pour séparer les amines préparées de l'au moins un catalyseur présent pendant l'hydrogénation.

7. Utilisation d'un système qui comprend
un réacteur d'hydrogénation (2),
un premier dispositif d'agitation (3) agencé dans le premier réacteur (2),
une entrée (8) pour les composés nitroaromatiques agencée dans le premier réacteur (2),
une entrée (9) pour l'hydrogène agencée dans le premier réacteur (2),
un réacteur de polissage (13), ayant au plus 1/20 du volume du réacteur d'hydrogénation (2),
un dispositif de raccordement (11), reliant lesdits réacteurs d'hydrogénation et de polissage (2, 13), qui est une entrée pour ledit réacteur de polissage (13), et une sortie pour ledit réacteur d'hydrogénation (2),
dans laquelle le dispositif de raccordement (11) est la seule entrée pour ledit réacteur de polissage (13) et est agencé en position adjacente ou à proximité d'un niveau de liquide gazéifié des réacteurs (2, 13)
pour la préparation d'amines par hydrogénation de nitrocomposés aromatiques en phase liquide selon la revendication 1.

8. Utilisation selon la revendication 7, dans laquelle le réacteur de polissage (13) comprend des agencements pour la circulation et la distribution de gaz.

9. Utilisation selon les revendications 7 ou 8, dans laquelle le réacteur de polissage (13) comprend un dispositif d'agitation (14) qui comporte un arbre d'agitation creux (16), étant agencé pour recirculation et distribution de gaz.

10. Utilisation selon les revendications 7 ou 8, dans laquelle le réacteur de polissage (13) comprend une conduite de recirculation pour la phase gazeuse (23) reliée à la partie supérieure du réacteur de polissage (13) et au-dessous d'un niveau de liquide gazéifié (12) du réacteur de polissage (13), de préférence à une position au-dessous du dispositif d'agitation (14).

11. Utilisation selon l'une quelconque des revendications 7 à 10, comprenant en outre un dispositif de dissociation (18) après le réacteur de polissage (13).

12. Utilisation selon l'une quelconque des revendications 7 à 11, comprenant en outre un filtre (20) .
